# EUROPÄISCHE PATENTSCHRIFT

(11) **EP 2 087 927 B1**
(45) Veröffentlichungstag und Bekanntmachung des Hinweises auf die Patenterteilung: **29.06.2011**
(21) Anmeldenummer: 09000182.7
(22) Anmeldetag: 09.01.2009
(51) Int. Cl.: A63B 23/18, A61M 16/00

(54) **Therapiegerät**
Therapy device
Appareil de thérapie

(30) Priorität: 08.02.2008 DE 102008008161
(43) Veröffentlichungstag der Anmeldung: 12.08.2009
(73) Patentinhaber: R. Cegla GmbH & Co. KG, 56410 Montabaur (DE)
(72) Erfinder: Cegla, Ulrich-Hartmann , Prof. Dr. med, 56410 Montabaur (DE)
(74) Vertreter: Engelhardt, Volker

(56) Entgegenhaltungen:
- EP-A- 0 681 853
- EP-A- 1 772 165

## Beschreibung

Die Erfindung bezieht sich auf ein Therapiegerät zur Verbesserung der Atmung eines Patienten, mit einem gekrümmt oder abgeknickt gestalteten Rohrstück, in dessen erste Stirnseite ein Mundstück eingesetzt ist.

Ein solches Therapiegerät zur Unterstützung der Atmung eines Patienten kann beispielsweise der EP 0681853 A1 entnommen werden. Dabei ist in das gekrümmt ausgestaltete Rohrstück in dessen erster Stirnseite ein Mundstück höhenverstellbar eingesetzt. Das Mundstück taucht somit bereichsweise in das Rohrstück ein. An dem eingetauchten Bereich des Mundstückes ist ein Schlauch angebracht, der durch die Innenkontur des Rohrstückes abgeknickt wird. Die zweite Stirnseite des Rohrstückes ist offen, so dass das Therapiegerät zum Ein- und Ausatmen eingesetzt werden kann. Durch die Abknickung des Schlauchstückes wird dieser beim Ein- und Ausatmen in eine oszillierende Schwingung versetzt, wodurch im Rachen- und Lungenraum des Patienten Schwingungen entstehen, durch die Erkrankungen der Atemwege behandelbar sind.

Das durch die EP 0681853 A1 offenbarte Therapiegerät hat sich zwar in der Praxis bewährt, jedoch hat sich herausgestellt, dass beim Einsaugen der Luft durch das Schlauchstück keine optimalen Behandlungserfolge erzielbar sind, da die von dem Schlauchstück erzeugten Schwingungen beim Einatmen in ihrer Amplitude und in ihrer Frequenz wesentlich geringer als beim Ausatmen ausfallen.

Des Weiteren ist bei diesem Therapiegerät beim Einatmen nachteilig, dass sich die Querschnittsfläche des Schlauchstückes oftmals derart verengt, dass der Luftwiderstand erheblich ist oder dass sogar das Schlauchstück vollständig verschlossen wird, wodurch keine Luft in die Atemwege des Patienten gelangt.

Es ist daher Aufgabe der Erfindung ein Therapiegerät der eingangs genannten Gattung derart auszugestalten, dass durch dieses beim Einatmen ein oszillierender Luftwiderstand erzeugbar ist, durch den die Atemwege des Patienten von Schleim und sonstigen Verunreinigungen befreit werden können oder durch den die Atemwege des Patienten trainiert werden können, um ein größeres Lungenvolumen für sportliche Höchstleistung zu erreichen. Das Therapiegerät soll dabei einfach zu handhaben und auf die individuellen medizinischen Bedürfnisse des Patienten einstellbar sein.

Diese Aufgabe wird dadurch gelöst, dass in die zweite Stirnseite des Rohrstückes ein mit diesem fest verbundener Haltezapfen angeordnet ist, der ganz oder teilweise in das Innere des Rohrstückes eintaucht und in den ein Durchlasskanal eingearbeitet ist, und dass an dem Haltezapfen ein biegsamer Schlauch angebracht ist, der im Inneren des Rohrstückes verläuft und dessen freies Ende im Bereich des Mundstückes zwischen der Innenwand des Rohrstückes frei beweglich ist.

Es ist besonders vorteilhaft, wenn in die zweite Stirnseite des Rohrstückes eine Schraube eingesetzt ist, die relativ zu dem Rohrstück drehbar an diesem abgestützt ist, und wenn in die Schraube eine Durchgangsbohrung eingearbeitet ist, in die der Haltezapfen eingesetzt ist, denn dadurch kann der an dem Haltezapfen angebrachte Schlauch in seiner Position innerhalb des Rohrstückes verändert werden. Da nämlich das Rohrstück gekrümmt ausgestaltet ist und folglich der Schlauch zumindest in der Grundstellung an zwei Positionen an der Innenwand des Rohrstückes anliegt, kann durch Verdrehen der Schraube eine Veränderung der Anlagefläche zwischen der Innenwand des Rohrstückes und des Schlauches oder eine Torsion des Schlauches erreicht werden. Solche Einstellungsmöglichkeiten führen vorteilhafterweise zu einer Anpassung des Luftwiderstandes, denn die durch das Mundstück eingesaugte Luft fließt zunächst durch den Schlauch und wird von diesem stoßweise abgegeben. Durch die einstellbare Positionierung des Schlauches kann nämlich die Knickung des Schlauches verändert werden, wodurch der erzielte Luftwiderstand von den Knickverhältnissen des Schlauches abhängt, so dass jeder Patient den Luftwiderstand des Schlauches an seine individuellen medizinischen Bedürfnisse anpassen kann.

Eine solche Einstellung des Luftwiderstandes kann auch dadurch erreicht werden, dass die in das Rohrstück eintauchende Länge des Haltezapfens variabel einstellbar ist und somit der Schlauch in unterschiedlichen Höhenlagen im Inneren des Rohrstückes verläuft.

Weitere vorteilhafte Weiterbildungen der Erfindung ergeben sich aus den Unteransprüchen.

In der Zeichnung sind drei erfindungsgemäße Therapiegeräte im Schnitt dargestellt, die nachfolgend näher erläutert werden. Im Einzelnen zeigt:
- Figur 1: ein erstes Ausführungsbeispiel eines Therapiegerät mit einem gekrümmt ausgestalteten Rohrstück, in dessen Inneren ein an einem Haltezapfen angebrachter Schlauch anliegt, dessen freies Ende im Bereich eines Mundstückes zwischen der Innenwand des Rohrstückes frei beweglich ist, in einem ersten Betriebszustand,
- Figur 2: das Therapiegerät gemäß Figur 1 in einem zweiten Betriebszustand,
- Figur 3: das Therapiegerät gemäß Figur 1 in einem dritten Betriebszustand,
- Figur 4: das Therapiegerät gemäß Figur 1 in einem vierten Betriebszustand,
- Figur 5: ein zweites Ausführungsbeispiel eines Therapiegerätes mit einem gekrümmten Rohrstück, in das ein Entlüftungsrohr im Bereich des Mündstückes eingesetzt ist und durch das Umgebungsluft einsaugbar ist, und
- Figur 6: ein drittes Ausführungsbeispiel eines Therapiegerätes mit einem gekrümmten Rohrstück und einen in dieses eingesetzten Haltezapfen, der von einem Deckel nach außen nahezu luftdicht verschlossen ist und durch den die Luftzufuhr einstellbar ist.

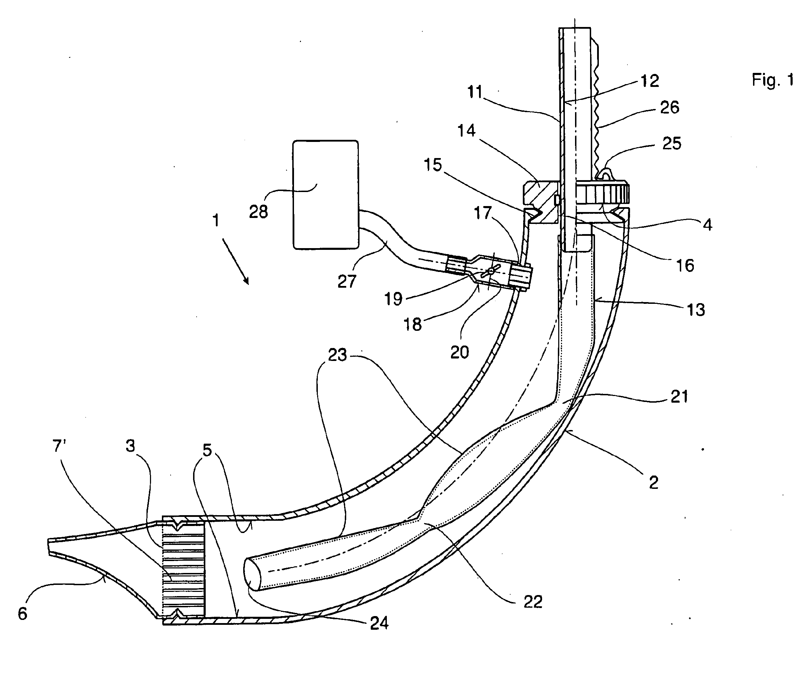

Aus den Figuren 1 bis 4 kann ein Therapiegerät 1 entnommen werden, durch das die Atemwege eines Patienten mittels eines oszillierende Luftwiderstandes behandelbar sind. Das Therapiegerät 1 dient dabei zur Unterstützung der Atmung beim Einatmen des Patienten oder als Sportgerät, um die Lungeneinsaugleistung bzw. das Lungenvolumen des Sportlers zu erhöhen.

Das Therapiegerät 1 besteht aus einem gekrümmt ausgebildeten Rohrstück 2, dessen Längsschnitt in etwa einem Viertelkreis nachgebildet ist. Das Rohrstück 2 weist zwei offen ausgebildete Stirnseiten 3 und 4 auf. In die erste Stirnseite 3 ist ein Mundstück 6 eingeschoben, das als Schnabel ausgebildet ist. Im Inneren des Mundstückes 6 ist mindestens ein Filtereinsatz 7' oder 7" vorgesehen. Der Filtereinsatz 7' ist dabei zu dem freien Enden 24 des Schlauches 13 benachbart angeordnet und weist mikroporöse Durchgangsöffnungen auf, durch die Verunreinigungen oder sonstige Partikel aufgefangen werden sollen, die in der einzuatmenden Luft vorhanden sind oder die sich vom Schlauch 13 lösen. Der Filtereinsatz 7" ist mit medizinischen Substraten oder anderen Stoffen, die für die Atemwege des Patienten eine heilsame Wirkung aufweisen, getränkt, so dass beim Einatmen die Luft den Filtereinsatz 7" durchströmt und folglich partikelweise das medizinische Substrat aufnimmt und in die Atemwege des Patienten mit jedem Atemzug abgibt. In den Figuren 1 bis 4 sind die verschiedenen Wahlmöglichkeiten dargestellt; in dem Mundstück 6 sind nämlich die andersartigen Filtereinsätze 7' bzw. 7" eingebraut.

An der zweiten Stirnseite 4 des Rohstückes 2 ist eine Schraube 14 in einem Gewinde 15 drehbar abgestützt. In das Zentrum der Schraube 14 ist eine Durchgangsbohrung 16 eingearbeitet, in der ein Haltezapfen 11 angeordnet und dort höhenverstellbar gehalten ist. Der Haltezapfen 11 verläuft demnach teilweise im Inneren des Rohrstückes 2. Durch den Haltezapfen 11 wird ein auf diesen aufgeschobener Schlauch 13, dessen konstruktiver Aufbau nachfolgend näher erläutert wird im Inneren des Rohrstückes 2, fixiert. In den Haltezapfen 11 ist ein Durchlasskanal 12 eingearbeitet, der in das Innere des Schlauches 13 einmündet. Die Umgebungsluft wird demnach beim Einatmen durch den Durchlasskanal 12 in den Schlauch 13 und von dort in das Mundstück 6 eingesaugt.

An der Außenseite des Haltezapfens 11 sind eine Vielzahl von Kerben 26 eingeformt, die in Form von Hinterschneidungen ausgebildet sind. An der Außenseite der Schraube 14 ist ein mit dieser verbundener Rasthaken 25 angeordnet, der in die jeweilige Kerbe 26 eingreift, wodurch der Haltezapfen 11 mittels des Rasthakens 25 an der Schraube 14 höhenverstellbar gehalten ist.

Der Schlauch 13 weist mindestens zwei Knickbereiche 21 bzw. 22 auf, die als Übergangsbereich zwischen einem Eingangsbereich des Schlauchstückes 13 und zwei bauchförmigen Teilbereichen 23 des Schlauches 13 angeordnet sind. Die Innendurchmesser der Knickbereiche 21 und 22 sind dabei konstruktiv kleiner ausgebildet als der Innendurchmesser der beiden bauchigen Teilbereiche 23 des Schlauches 13. Durch Drehen der Schraube 14 kann somit die Position des Schlauches 13 gegenüber der Wand 5 des Rohrstückes 2 verändert werden und dieser tortiert um die eigene Längsachse. In Figur 1 verlaufen die Knickbereiche 21 und 22 im Bereich der Wand 5 des Rohrstückes 2. Diese können von der Wand 5 durch Verdrehen der Schraube 14 abgehoben werden. Durch die Höhenverstellung des Haltezapfens 11 kann der im Inneren des Rohrstücks 2 verlaufende Schlauch 13 in seiner Position verändert werden, wodurch der Verlauf des Schlauches 13 gegenüber der Wand 5 des Rohrstücks 13 einstellbar ist.

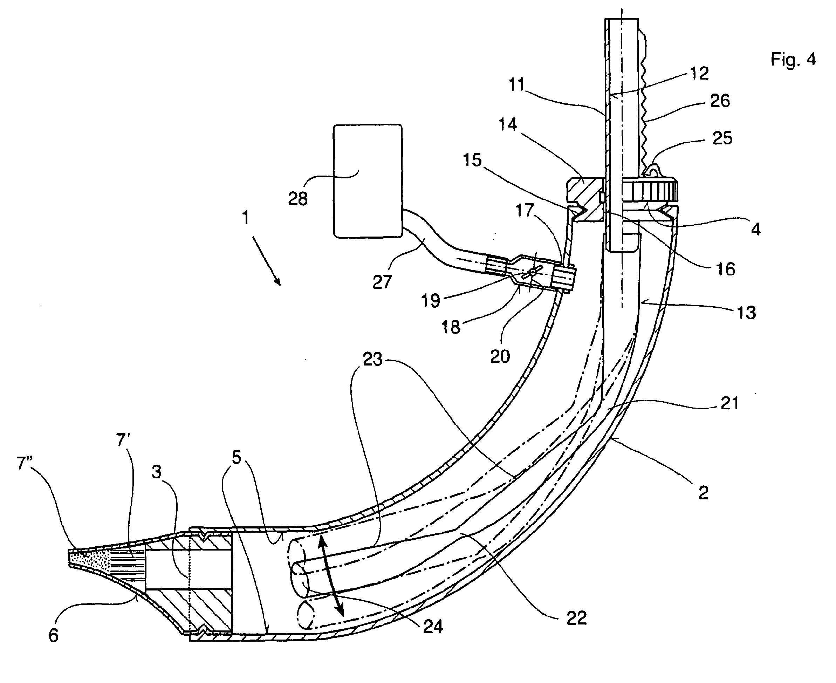

Beim Einatmen wird nämlich das freie Ende 24 des Schlauches 13 in Schwingung versetzt, denn dieses kann zwischen der Wand 5 frei hin und her flattern. Des Weiteren werden einzelnen Teilbereiche des Schlauches 13 unterschiedlich mit Luft während des Einatemvorganges gefüllt. Durch die Knickbereiche 21 und 22 wird nämlich das Ausströmen der Luft aus dem jeweiligen Teilbereich 23 verlangsamt, wodurch der vorgeschaltete Teilbereich 23 in Form eines Bauches aufgeblasen wird. Dabei ist Figur 4 zu entnehmen, wie das freie Ende 24 des Schlauches 13 zwischen der Wand 5 hin und her bewegt wird.

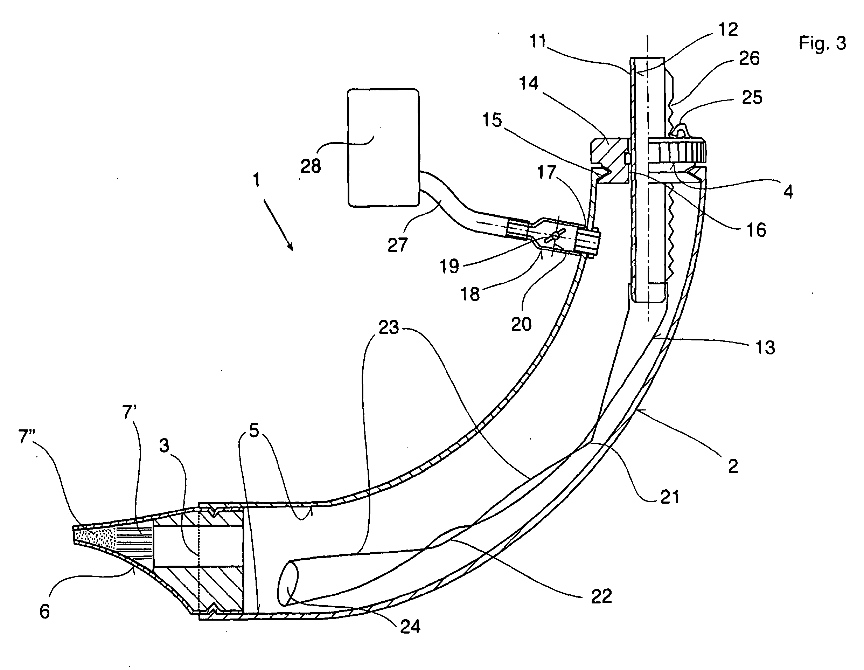

Der Schlauch 13 in Figur 3 weist vier Teilbereiche 23 auf, deren Längenabmessung unterschiedlich groß bemessen ist. Die Übergangsbereiche zwischen den bauchig ausgestalteten Teilbereichen 23 sind durch konstruktive Maßnahmen bereits verengt bzw. die Knickungen des Schlauches 13 werden durch Verdrehen der Schraube 14 oder durch die Höhenveränderung des Haltezapfens 11 erzeugt. Eine solche Verengung im Übergangsbereich 22 von zwei benachbarten Teilbereichen 23 kann auch durch die Anlage des Schlauches 13 an der Wand 5 des Rohrstücks 2 erreicht werden.

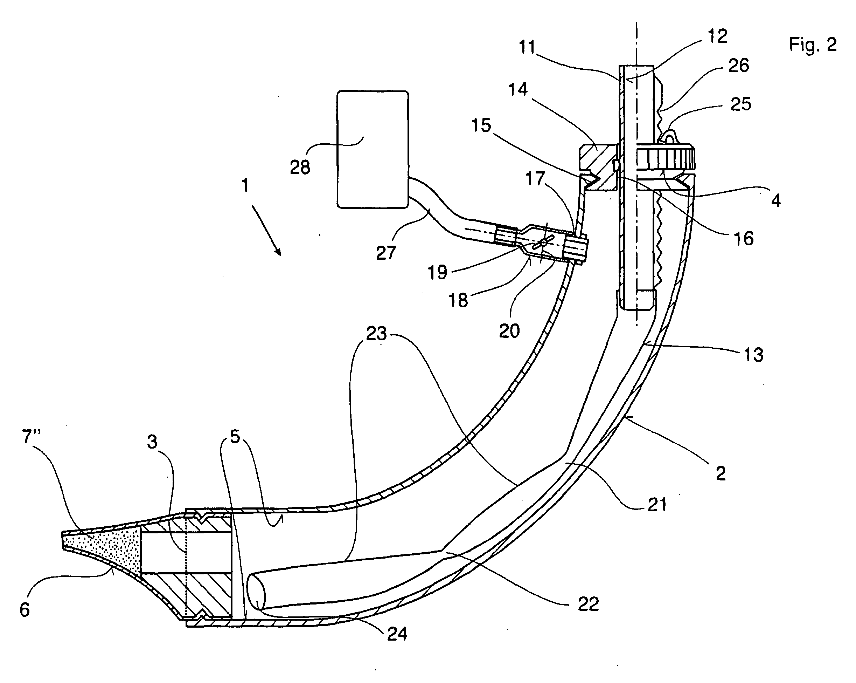

In Figur 2 ist der Haltezapfen 11 gegenüber der Position in Figur 1 tiefer in das Rohrstück 2 eingeschoben, wodurch der Schlauch 13 in eine andersartige Position und einer damit verbundenen Auslenkung gegenüber der Figur 1 überführt wird.

In die Wand 5 des Therapiegerätes 1 ist eine Einlassöffnung 17 eingearbeitet, in die ein Einlassventil 18 eingeschoben ist, das im Bereich des Haltezapfens 11 verläuft und das über einen Schlauch 27 mit dem Inneren eines Behälters 28 verbunden ist. In dem Einlassventil 18 ist ein Stellglied 19 vorgesehen, das von außen bedienbar ist. Durch das Verstellglied 19 wird die durchströmte Querschnittsfläche 20 des Einlassventils 18 verändert, so dass der Patient nicht nur über den Durchlasskanal 12 und den Schlauch 13, sondern auch über das Einlassventil 18 und dessen durchströmte Querschnittsfläche 20 Luft aus der Umgebung oder ein Gas bzw. Medizin einsaugen kann. Das Gas oder die Medizin, vorzugsweise Sauerstoff, wird dabei in den Behälter 28 gelagert, dessen Inneres mit den Einlassventil 18 verbunden ist und somit in einer voreinstellbaren Menge in das Innere des Rohrstückes 2 gelangt. Das im Behälter 28 vorhandene Gas kann unter Überdruck stehen oder wird aus diesem durch Einatmen des Patienten angesaugt.

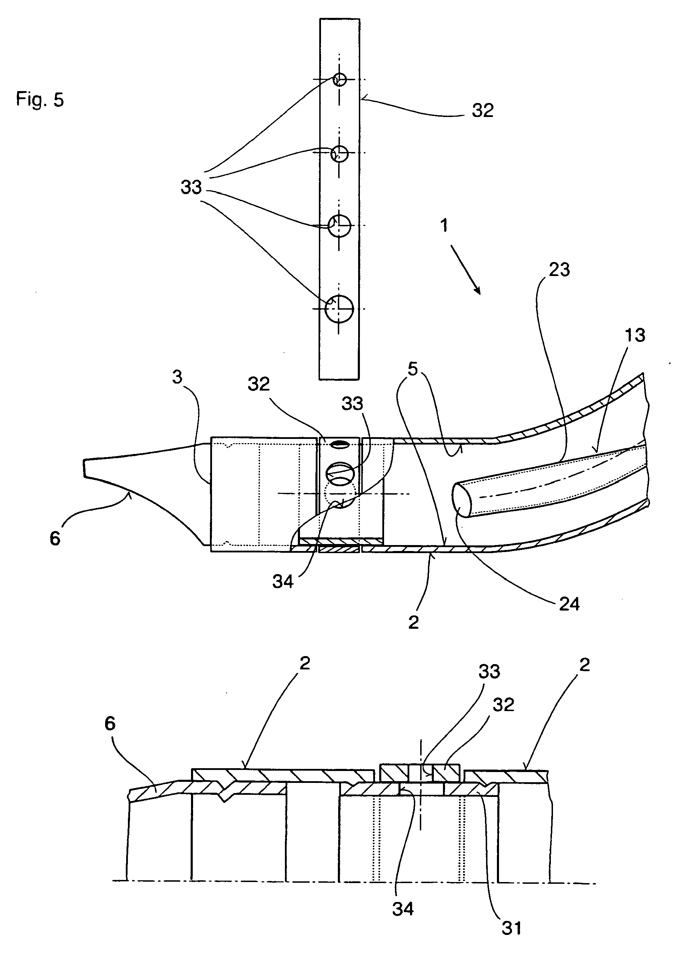

Aus Figur 5 ist ein zweites Ausführungsbeispiel in Form einer Weiterbildung des Therapiegerätes 1 nach den Figuren 1 bis 4 zu entnehmen. Zwischen dem Mundstück 6 und dem freien Ende 24 des Schlauches 13 ist nämlich ein Entlüftungsrohr 31 eingesetzt, durch das eine mit der Umgebungsluft kommunizierende Bypassleitung zur Verfügung steht. In das Entlüftungsrohr 31 ist eine Einlassbohrung 34 mit einem vorgegebenen Innendurchmesser eingearbeitet. Die Längsachse der Einlassbohrung 34 verläuft senkrecht zu der Strömungsrichtung der Atemluft im Inneren des Rohrstückes 2. Um eine voreinstellbare Zufuhr von Umgebungsluft durch die Einlassbohrung 34 zu ermöglichen, ist diese mit einem Stellring 32 verschlossen. Der Stellring 32 ist auf dem Außenumfang des Entlüftungsrohres 31 drehbar gelagert und in diesem sind gemäß der in der Figur 5 gezeigten Abwicklung vier Durchgangsöffnungen 33 eingearbeitet. Die vier Durchgangsöffnungen 33 weisen einen unterschiedlich groß bemessenen Innendurchmesser auf; der größte Innendurchmesser der Durchgangsöffnung 33 entspricht dabei dem Innendurchmesser der Einlassbohrung 34. Wird nunmehr die Einlassbohrung 34 durch den Stellring 32 verschlossen, strömt durch die Einlassbohrung 34 keine Umgebungsluft und die Wirkungsweise des Therapiegerätes 1 entspricht der Grundvariante des Therapiegerätes 1 nach den Figuren 1 bis 4.

Durch Verdrehen des Stellringes 32 können nunmehr die vier in diesem eingearbeiteten Durchgangsöffnungen 33 fluchtend zu der Einlassbohrung 34 gebracht werden, so dass in Abhängigkeit von der oberhalb der Einlassbohrung 34 angeordneten Durchgangsöffnung 33 eine vorgegebene Luftmenge in das Innere des Rohrstückes 2 während des Einatemvorganges eingesaugt werden kann. Durch diese Maßnahme wird erreicht, dass der Patient eine erhöhte Lungenkraft aufzubringen hat, um das freie Ende 24 des Schlauches 13 in Schwingung zu versetzen, denn zusätzlich zu der durch den Schlauch 13 einzusaugenden Luftmenge gelangt über die Durchgangsöffnungen 33 und die Einlassbohrung 34 Umgebungsluft in das Innere des Rohrstückes 2. Insbesondere diese zusätzliche Möglichkeit Umgebungsluft einzuatmen, kann als medizinischer Beitrag zur Steigerung des Lungenvolumens und der Lungenkraft angesehen werden, so dass das Therapiegerät 1 nach Figur 5 auch als Sportgerät, beispielsweise für Taucher oder Radfahrer, verwendet werden kann.

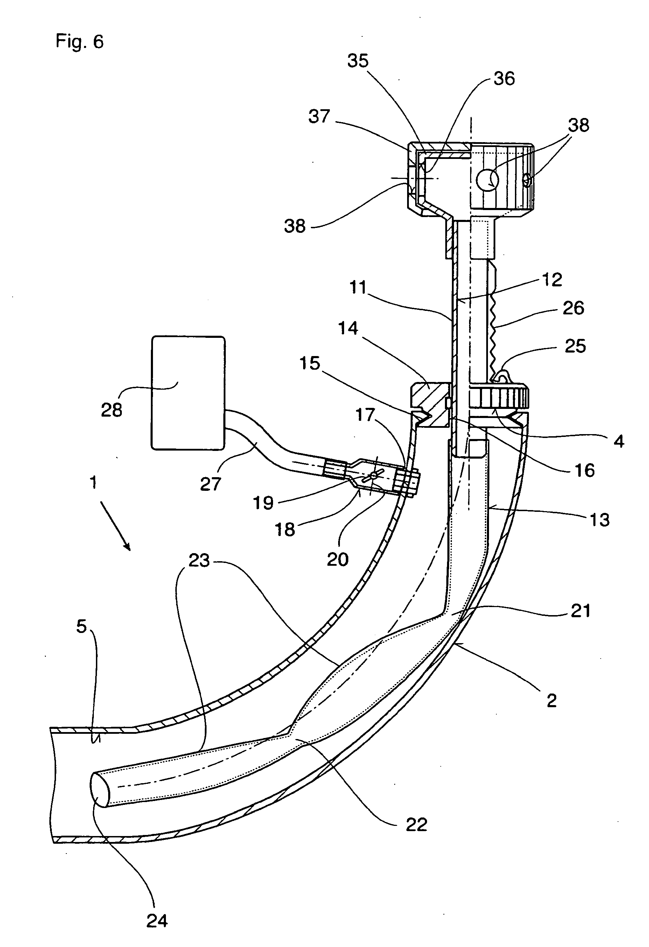

In Figur 6 ist eine dritte Weiterbildung des Therapiegerätes 1 nach den Figuren 1 bis 4 abgebildet. Das freie Ende des Haltezapfens 11 ist nämlich mit einem Deckel 35 verschlossen, in dem eine Einlassbohrung 36 eingearbeitet ist. Die Einlassbohrung 36 wird durch einen an dem Deckel 35 drehbar abgestützten Stellring 37 überdeckt. In die seitliche Außenkontur des Stellringes 37 sind dabei mehrere Durchlasskanäle 38 eingearbeitet, deren Innendurchmesser zueinander unterschiedlich groß ausgestaltet sind. Der größte Innendurchmesser der Einlasskanäle 38 entspricht dabei, dem Innendurchmesser der Einlassbohrung 36. Durch Verdrehen des Stellringes 37 kann somit wahlweise die Durchflussmenge der Umgebungsluft, die durch die Einlassbohrung 36 und den jeweiligen Durchlasskanal 38 gelangt, eingestellt werden. In Abhängigkeit der ausgewählten Einstellung wird eine größere Lungenkraft benötigt, um aus der Umgebung Luft durch die Einlassbohrung 34 in den Schlauch 13 einzusaugen. Auch diese Maßnahme erhöht somit die Wirkung des Therapiegerätes 1 in der Form, dass dieses als Sportgerät zu Trainingszwecken für Hochleistungssportler eingesetzt werden kann.

Die in den Figuren 5 und 6 dargestellten und erläuterten Weiterbildungen des Therapiegerätes 1 nach den Figuren 1 bis 4 können miteinander kombiniert werden oder als modularer Aufbau wahlweise an das Therapiegerät 1 nach den Figuren 1 bis 4 angeschlossen werden. Durch die in den Figuren 5 und 6 beschriebenen Maßnahmen wird die Sauggeschwindigkeit verändert, durch die die Schwingungen des Schlauches 13 ausgelöst werden. Jeder Patient kann somit das Therapiegerät 1 auf seine speziellen Bedürfnisse einstellen und dieses als so genannte incentive Spirometrie bzw. inspiratoischer Muskeltrainer einsetzen. Durch auf den Haltezapfen 11 aufgesteckte System bestehend aus Deckel 35 und Stellring 37, die gemeinsam eine Lochkranzvorrichtung bilden, wird der benötigte Einatemdruck eingestellt und entsprechend variiert, indem der Stellring 37 in verschieden Positionen verdreht wird.

## Patentansprüche

1. Therapiegerät (1) zur Verbesserung der Atmung eines Patienten, mit einem gekrümmt oder abgeknickt gestalteten Rohrstück (2), in dessen erste Stirnseite (3) ein Mundstück (6) eingesetzt ist,
**dadurch gekennzeichnet,**
**dass** in die zweite Stirnseite (4) des Rohrstückes (2) ein mit diesem fest verbundener Haltezapfen (11) angeordnet ist, und in den ein Durchlasskanal (12) eingearbeitet ist, der ganz oder teilweise in das Innere des Rohrstückes (2) eintaucht und dass an dem Haltezapfen (11) ein biegsamer Schlauch (13) angebracht ist, der im Inneren des Rohrstückes (2) verläuft und dessen freies Ende (24) im Bereich des Mundstückes (6) zwischen der Innenwand (5) des Rohrstückes (2) frei beweglich ist.

2. Therapiegerät nach Anspruch 1,
**dadurch gekennzeichnet,**
**dass** in die zweite Stirnseite (4) des Rohrstückes (2) eine Schraube (14) eingesetzt ist, die relativ zu dem Rohrstück (2) drehbar an einem Gewinde (15) abgestützt ist, und dass in die Schraube (14) eine Durchgangsbohrung (16) eingearbeitet ist, in die der Haltezapfen (11) einsetzbar ist.

3. Therapiegerät nach Anspruch 1 oder 2,
**dadurch gekennzeichnet,**
**dass** die in das Rohrstück (2) eintauchende Länge des Haltezapfens (11) variabel einstellbar ist.

4. Therapiegerät nach Anspruch 2 oder 3,
**dadurch gekennzeichnet,**
**dass** an der Außenseite der Schraube (14) ein Rasthaken (25) angeformt ist, der in an der Außenseite des Haltezapfens (11) eingearbeitete Kerben (26) in Form von Hinterschneidungen eingreift, und dass durch den Rasthaken (25) der Haltezapfen (11) höhenverstellbar an der Schraube (14) gehalten ist.

5. Therapiegerät nach einem oder mehreren der vorgenannten Ansprüche,
**dadurch gekennzeichnet,**
**dass** in die Wand (5) des Rohrstückes (2) eine Einlassöffnung (17), vorzugsweise im Bereich des Haltezapfens (11) eingearbeitet ist, in die ein Einlassventil (18) eingesetzt ist.

6. Therapiegerät nach Anspruch 5,
**dadurch gekennzeichnet,**
**dass** in dem Einlassventil (18) ein Verstellglied (19) vorgesehen ist, durch das die durchströmte Querschnittsfläche (20) des Einlassventiles (18) veränderbar ist.

7. Therapiegerät nach Anspruch 5 oder 6
**dadurch gekennzeichnet,**
**dass** an das Einlassventil (18) ein Behälter (28) angeschlossen ist, und dass im Inneren des Behälters (28) ein Gas oder ein Gas- Flüssigkeitsgemisch, vorzugsweise Sauerstoff oder Medizin, vorhanden ist, das durch das Einlassventil (18) in einer voreinstellbaren Menge in das Innere des Rohrstückes (2) mittels Überdruck einströmt oder mittels der Atmung des Patienten einsaugbar ist.

8. Therapiegerät nach einem oder mehreren der vorgenannten Ansprüche,
**dadurch gekennzeichnet,**
**dass** der Schlauch (13) im Ruhezustand mindestens an einer Stelle der Wand (5) des Rohrstückes (2) anliegt und durch die Innenkontur des Rohrstückes (2) abgeknickt ist.

9. Therapiegerät nach einem oder mehreren der vorgenannten Ansprüche,
**dadurch gekennzeichnet,**
**dass** der Schlauch (13) mindestens zwei Teilbereiche (23) aufweist, die miteinander durch einen Übergangs- oder Knickbereich (22) verbunden sind, und dass der Innendurchmesser des Übergangsbereiches (22) kleiner bemessen ist, als der Innendurchmesser der beiden benachbarten Teilbereiche (23) des Schlauches (13).

10. Therapiegerät nach einem oder mehreren der vorgenannten Ansprüche,
**dadurch gekennzeichnet,**
**dass** in das Mundstück (6) mindestens ein Filtereinsatz (7', 7") einsteckbar ist und dass durch den Filtereinsatz (7') die eingesaugte Luft von Verunreinigungen oder Partikeln gereinigt ist und dass der Filtereinsatz (7") mit einer Medizin, einem Substrat oder dgl. getränkt ist, die beim Einatmen in die Atemwege des Patienten gelangen.

11. Therapiegerät nach einem oder mehreren der vorgenannten Ansprüche,
**dadurch gekennzeichnet,**
**dass** zwischen dem freien Enden (24) des Schlauches (13) und dem Mundstück (6) ein Entlüftungsrohr (31) in das Rohrstück (2) eingesetzt ist, dass das Entlüftungsrohr (31) eine Einlassbohrung (34) aufweist, deren Längsachse senkrecht zu der Strömungsrichtung der eingesaugten Atemluft verläuft, und dass an dem Außenumfang des Entlüftungsrohres (31) ein Stellring (32) drehbar gehalten ist, in den ein oder mehrere Durchgangsöffnungen (33) mit unterschiedlich groß bemessenen Innendurchmessern eingearbeitet sind, die fluchtend zu der Einlassbohrung (34) angeordnet sind.

12. Therapiegerät nach einem oder mehreren der vorgenannten Ansprüche,
**dadurch gekennzeichnet,**
**dass** das nach außen abstehende freie Ende des Haltezapfens (11) mit einem Deckel (35) verschlossen ist, dass in dem Deckel (35) eine Einlassbohrung (36) eingearbeitet ist, die von einem an dem Deckel (35) drehbar gelagerten Stellring (37) abgedeckt ist und dass in dem Stellring (37) eine Vielzahl von Durchlasskanälen (38) vorgesehen sind, fluchtend zu der Einlassbohrung (36) angeordnet sind und die unterschiedlich groß bemessene Innendurchmesser aufweisen.

## Claims

1. A therapy device (1) for improving the respiration of a patient, with a curved or bent pipe section (2) and a mouthpiece (6) inserted in its first end (3),
**characterized in that,**
a holding peg (11) that is firmly connected to the pipe section (2) can be pushed into the second end (4) of the pipe section (2), and has a passage channel (12) worked into it that penetrates inside the pipe section (2) completely or in part and that the holding peg (11) has a flexible hose (13) attached to it that runs inside the pipe section (2) and the free end (24) of which can move freely in the area of the mouthpiece (6) between the inner wall (5) of the pipe section (2).

2. The therapy device in accordance with Claim 1,
**characterized in that,**
a screw (14) is inserted in the second end (4) of the pipe section (2), which can be turned in relation to the pipe section (2) and is supported on a thread (15), and that the screw (14) has a passage hole (16) worked into it, in which the holding peg (11) can be inserted.

3. The therapy device in accordance with Claim 1 or 2,
**characterized in that,**
the length of the holding peg (11) that penetrates into the pipe section (2) is variably adjustable.

4. The therapy device in accordance Claim 2 or 3,
**characterized in that,**
the outside of the screw (14) has a detent hook (25) formed onto it, which engages in notches (26) in the form of undercuts worked into the outside of the holding peg (11), and that the detent hook (25) holds the holding peg (11) on the screw (14) so it is adjustable in height.

5. The therapy device in accordance with one or more of the aforementioned claims,
**characterized in that,**
an inlet opening (17) is worked into the wall (5) of the pipe section (2), preferably in the area of the holding peg (11), with an inlet valve (18) inserted in the inlet opening (17).

6. The therapy device in accordance with Claim 5,
**characterized in that,**
an adjusting element (19) is provided in the inlet valve (18), by means of which the cross-sectional area (20) of the inlet valve (18) through which air flows can be changed.

7. The therapy device in accordance with Claim 5 or 6,
**characterized in that,**
a container (28) is connected to the inlet valve (18), and that the container (28) contains a gas or a gas/liquid mix, preferably oxygen or medicine, which flows through the inlet valve (18) in an adjustable quantity into the inside of the pipe section (2) by means of positive pressure or can be sucked in by means of the patient's respiration.

8. The therapy device in accordance with one or more of the aforementioned claims,
**characterized in that,**
when at rest, the hose (13) makes contact with the wall (5) of the pipe section (2) at least at one point, and is bent by the inner contour of the pipe section (2).

9. The therapy device in accordance with one or more of the aforementioned claims,
**characterized in that,**
the hose (13) has at least two subsections (23) that are connected together by a transitional or bent area (22), and that the inner diameter of the transitional area (22) is smaller in size than the inner diameter of the two adjacent subsections (23) of the hose (13).

10. The therapy device in accordance with one or more of the aforementioned claims,
**characterized in that,**
at least one filter insert (7', 7") can be inserted into the mouthpiece (6) and that the filter insert (7') cleans the sucked-in air of impurities or particles and that the filter insert (7") is impregnated with a medicine, a substrate or the like which penetrate the patient's airways during inhalation.

11. The therapy device in accordance with one or more of the aforementioned claims,
**characterized in that,**
a ventilation tube (31) is inserted in the pipe section (2) between the free end (24) of the hose (13) and the mouthpiece (6), that the ventilation tube (31) has an inlet hole (34) with its lengthways axis running vertical to the direction of flow of the sucked-in respiratory air, and that the outer profile of the ventilation tube (31) has an adjusting ring (32) held on it in a rotational arrangement, into which one or more passage openings (33) with differently sized inner diameters are worked which are flush with the inlet hole (34).

12. The therapy device in accordance with one or more of the aforementioned claims,
**characterized in that,**
the free end of the holding peg (11) projecting outwards is sealed with a cover (35), that the cover (35) has an inlet hole (36) worked into it which is covered by an adjusting ring (37) in a rotating mounting on the cover (35) and that there is a plurality of passage channels (38) provided in the adjusting ring (37) which are arranged flush with the inlet hole (36) and have inner diameters of differing sizes.

## Revendications

1. Appareil de thérapie (1) servant à améliorer la respiration d'un patient, avec une pièce tubulaire (2) courbée ou plié, dans la face frontale (3) de laquelle il est inséré un embouchoir (6),
**caractérisé en ce que**
dans la deuxième face frontale (4) de la pièce tubulaire (2), il est prévu un téton de retient solidaire (11) dans lequel il est pratiqué un canal de passage (12) entrant partiellement ou complètement dans l'intérieur de la pièce tubulaire (2), et que sur le téton de retient (11), il est fixé un tuyau flexible (13) s'étendant à l'intérieur de la pièce tubulaire (2) et dont l'extrémité libre (24) peut se déplacer librement au niveau de l'embouchoir (6) entre la paroi intérieure (5) de la pièce tubulaire (2).

2. Appareil de thérapie d'après la revendication 1,
**caractérisé en ce que**
dans la deuxième face frontale (4) de la pièce tubulaire (2), il est prévu une vis (14) qui, relativement par rapport à la pièce tubulaire (2) est appuyée en rotation sur un filetage (15), et que dans la vis (14) il est pratiqué un alésage de passage (16) dans lequel se laisse insérer le téton de retient (11).

3. Appareil de thérapie d'après les revendications 1 ou 2,
**caractérisé en ce que**
la longueur du téton de retient (11) s'enfonçant dans la pièce tubulaire (2) se laisse ajuster.

4. Appareil de thérapie d'après les revendications 2 ou 3,
**caractérisé en ce que**
sur la face extérieure de la vis (14), il est formé un crochet de crantage (25) qui s'engrène dans des encoches (26) sous la forme de contre-dépouilles, pratiquées sur la face extérieure du téton de retient (11), et que le crochet de crantage (25) permet de retenir le téton de retient (11) avec réglage en hauteur sur la vis (14).

5. Appareil de thérapie d'après une ou plusieurs des revendications précédentes,
**caractérisé en ce que**
dans la paroi (5) de la pièce tubulaire (2), de préférence au niveau du téton de retient (11), il est pratiqué une ouverture d'entrée (17), dans laquelle il est inséré une valve d'entrée (18).

6. Appareil de thérapie d'après la revendication 5,
**caractérisé en ce que**
dans la valve d'entrée (18), il est prévu un élément de réglage (19) permettant d'ajuster la section traversée (20) de la valve d'entrée (18).

7. Appareil de thérapie d'après les revendications 5 ou 6,
**caractérisé en ce**
**qu'**à la valve d'entrée (18), il est raccordé un réservoir (28) et qu'à l'intérieur du réservoir (28), il existe un gaz ou un mélange de gaz/liquide, de préférence de l'oxygène ou un médicament qui atteint par surpression en dosage réglé l'intérieur de la pièce tubulaire (2) et qui se laisse aspirer par la respiration du patient,

8. Appareil de thérapie d'après une ou plusieurs des revendications précédentes,
**caractérisé en ce que**,
en position de repos, le tuyau flexible (13) porte sur au moins un endroit de la paroi (5) de la pièce tubulaire (2) et est plié par le contour intérieur de la pièce tubulaire (2).

9. Appareil de thérapie d'après une ou plusieurs des revendications précédentes,
**caractérisé en ce que**
le tuyau flexible (13) comporte au moins deux sections (23) liées entre elles par un passage ou un endroit courbé (22), et que le diamètre intérieur du passage (22) est inférieur au diamètre intérieur des deux sections voisines (23) du tuyau flexible (13).

10. Appareil de thérapie d'après une ou plusieurs des revendications précédentes,
**caractérisé en ce que**
dans l'embouchoir (6), il se laisse insérer au moins un élément filtrant (7', 7"), que l'élément filtrant (7') épure l'air aspiré des pollutions ou des particules et que l'élément filtrant (7") est rempli d'un médicament, d'un substrat ou d'un produit similaire qui, à la respiration, entre dans la voie respiratoire du patient.

11. Appareil de thérapie d'après une ou plusieurs des revendications précédentes,
**caractérisé en ce que**,
entre les extrémités libres (24) du tuyau flexible (13) et de l'embouchoir (6), il est inséré dans la pièce tubulaire (2) un tube de dégazage (31) et que le tube de dégazage (31) comporte un alésage d'entrée (34) dont l'axe longitudinal s'étend perpendiculairement à la direction du flux de l'air aspiré et que sur le pourtour extérieur du tube de dégazage (31), il est retenu en rotation un anneau de réglage (32), dans lequel il est pratiqué une ou plusieurs ouvertures de passage (33) avec des diamètres intérieurs de dimensions différentes, et qui sont disposées à fleur de l'alésage d'entrée (34).

12. Appareil de thérapie d'après une ou plusieurs des revendications précédentes,
**caractérisé en ce que**
l'extrémité libre saillant vers l'extérieur du téton de retient (11) est fermée par un couvercle (35), que dans le couvercle (35), il est pratiqué un alésage d'entrée (36) recouvert par un anneau de réglage (37) retenu en rotation sur le couvercle (35) et que dans l'anneau de réglage (37), il est prévu une multitude de canaux de passage (38) à fleur de l'alésage d'entrée (36), qui ont des diamètres intérieurs de dimensions différentes.
